# EUROPÄISCHE PATENTANMELDUNG

(11) **EP 2 532 421 A1**
(43) Veröffentlichungstag der Anmeldung: **12.12.2012**
(21) Anmeldenummer: 11004778.4
(22) Anmeldetag: 10.06.2011
(51) Int. Cl.: B01J 20/28, B01D 61/36, B01D 69/14, B01D 71/02, B01D 71/32, C07C 45/78, C12P 7/06, D04H 13/00

(54) **Verbundmaterial aus fluorhaltigem Polymer, hydrophoben Zeolith-Partikeln und metallischem Werkstoff**

(71) Anmelder: Süd-Chemie AG, 80333 München (DE)
(72) Erfinder: Koch Achim, 85368 Moosburg (DE); Zavrel Michael, 81379 München (DE)
(74) Vertreter: HOFFMANN EITLE

(57) **Zusammenfassung**

Die Erfindung betrifft ein Verbundmaterial umfassend a) eine poröse Matrix aus einem fluorhaltigen Polymer mit einem Anteil von Tetrafluorethylen-Monomereinheiten von wenigstens 95 mol-% bezogen auf die gesamte Anzahl der Monomereinheiten, b) hydrophobe Zeolith-Partikel, welche in die Matrix eingebettet und von ihr umgarnt sind, und c) mindestens einen metallischen Werkstoff, die Verwendung des Verbundmaterials zur Adsorption von organischen Molekülen aus einem gasförmigen oder flüssigen Stoffgemisch enthaltend wenigstens eine organische Komponente und ein Verfahren zur Abtrennung von organischen Molekülen aus einem gasförmigen oder flüssigen Stoffgemisch.

## Beschreibung

### Gebiet der Erfindung

Die Erfindung betrifft ein Verbundmaterial bestehend aus einem fluorhaltigen Polymer, hydrophoben Zeolith-Partikeln, und einem metallischen Werkstoff mit verbesserten mechanischen und thermischen Eigenschaften zur Adsorption von organischen Molekülen aus einem wässrigen Fluid.

### Hintergrund der Erfindung

Die Trennung von flüssigen oder gasförmigen Stoffgemischen wird seit Jahrzehnten für eine Vielzahl von Anwendungen kommerziell bzw. industriell betrieben. Beispiele sind chromatographische analytische oder präparative Trennung (high pressure liquid chromatography, HPLC oder gas chromatography GC) mittels Festphasenextraktion (solid phase extraction, SPE), Trennung von Gasen oder Flüssigkeiten durch Pervaporation oder Dampfpermeation mit sog. MMMs (mixed matrix membranes), Trocknung von Gasen und Flüssigkeiten mit porösen hydrophilen Trockenmitteln und selektive Adsorptions-/Desorptionsprozesse an hydrophobem Zeolith. Da sich die Trockenmittel alleine, entweder in Pulver- oder Granulatform, für bestimmte Anwendungen unter Druck, mit hohen Druckgradienten oder mechanischen Belastungen nicht eignen würden, werden sie in der Regel auf Träger aufgebracht. Stand der Technik ist daher die Verwendung von Formkörpern, zur Maximierung der wirksamen Fläche bei möglichst kleiner Bauform. Dies sind in der Regel Membranen, die aus Verbünden sorptiv wirkender Materialien zusammen mit Polymeren aufgebaut werden.

Als Matrixpolymer hierfür eignet sich Polytetrafluorethylen (PTFE, Hersteller z.B. DuPont oder Dyneon - 3M) vor allem, weil es fibrillierbar, thermisch stabil, chemisch inert und hydrophob ist, d.h. es ist zu stabilen und hochflexiblen Faservliesen verarbeitbar, im Arbeitstemperaturbereich von -250 bis +260°C verwendbar, nimmt weder Wasser auf, noch ist es löslich; zudem ist PTFE gegenüber Säuren und Laugen weitgehend inert.

PTFE ist teilkristallin und oberhalb der Phasenübergangstemperatur von 19°C fibrillierbar, d.h. durch die Anwendung von Scherkräften auf PTFE-Pulver, -Granulate oder die in der Dispersion enthaltenen PTFE-Kugeln lassen sich die im Material enthaltenen Kristallite zu dünnen Filamenten abspulen (dieser Effekt ist oberhalb von 30°C noch besser zu beobachten; hier liegt der zweite Phasenübergang von PTFE). Diese im besten Fall nur wenige Moleküllagen dicken Filamente sind imstande, durch geeignete Verarbeitungstechnik große Mengen Füllstoff zu umgarnen, einzubetten und festzuhalten, wodurch hochgradig vernetzte, hochgefüllte PTFE-Füllstoff-Komposite erhalten werden. Darüber hinaus verhaken und verschlaufen die Polymerfasern bei der Scherung miteinander, was dem Kompositmaterial eine gewisse mechanische Stabilität verleiht. Die Verarbeitbarkeit zu Filmen und Formkörpern kann allerdings aufgrund hoher benötigter Verformungskräfte abhängig vom Füllstoff stark behindert sein, weshalb es sich durchgesetzt hat, soweit möglich Gleitmittel zu verwenden (Wasser, Alkohole, Erdöldestillate, Kohlenwasserstoffe und andere Lösungsmittel), die den Verarbeitungsprozess erleichtern, die Fibrillierung unterstützen und das vorzeitige Zerstören/Zerreißen der Fasern durch zu starke Scherung unterbinden sollen. Nach der Formgebung wird das eingearbeitete Lösungsmittel in der Regel durch Erhitzen entfernt, wodurch ein zusätzlicher definierter Grad an Porosität zurückbleibt. Durch einen optionalen Sinterprozess des PTFE-Materials bei Temperaturen um 330°C, aber unter 360°C (beginnende Zersetzung) erhält das Kompositmaterial seine endgültige Stabilität und Formgebung.

Die Einbettung von Füllstoffen in fibrilliertes PTFE ist bekannt und wird in verschiedenen Patentdokumenten beschrieben.

Je nach Art des beschriebenen Zeolithen lassen sich die Patentdokumente des Stands der Technik wie folgt einteilen: JP 04048914 beschreibt die Herstellung eines Films mit feuchtigkeitsentziehender Funktion bestehend aus 1-10 Teilen fibrillierbarem Fluorpolymer (i.e. PTFE) und 100 Teilen feuchtigkeitsabsorbierendem Füller (Calciumchlorid, Zeolith, Aluminiumoxid oder Silica) mit Partikelgrößen <50µm, hergestellt durch Kneten und Herstellung von Folien. Optional wird ein Hilfsmittel eingesetzt (i.e. Wasser, Alkohol), welches den Fibrillierungsprozess unterstützt.

EP 1396175 beschreibt einen selbststützenden, Feuchtigkeit absorbierenden Film aus ultrahoch-molekularem (UHMW-) Polymer (MW>1.000.000 g/mol) mit eingebettetem Trockenmittel zum Schutz von elektrolumineszierenden Elementen vor Feuchtigkeit.

JP 63028428 beschreibt ein Trockenmittel bestehend aus PTFE, Zeolith und flüssigem Gleitmittel, welches durch Verkneten und Ausrollen in Formkörper überführt wird. Darüber hinaus wird ein hygroskopisches Metall- oder Alkalimetall-Salz (z.B. Lithiumchlorid) aus seiner ethanolischen Lösung auf der Oberfläche der Zeolithpartikel abgeschieden und das Material anschließend getrocknet. Es wird auch beschrieben, dass mit Vertiefungen versehene gerollte Folien Dampf in Längsrichtung passieren lassen können.

EP 0316159 beschreibt die Herstellung von Formkörpern zu Katalyse- oder Absorptionszwecken aus PTFE-Pulver oder -Dispersion und sorptiv wirksamem Füllstoff im Verhältnis 1:0.5-10 ggf. unter Verwendung eines flüssigen Gleitmittels, durch Vermischen der Komponenten zu einer Paste und anschließendem Sintern bei >327°C, wodurch ein selbsttragender Formkörper mit Oberfläche >50 m²/g entsteht.

WO 2005/049700 beschreibt eine Methode zur Herstellung von PTFE-Füllstoff-Kompositen basierend auf aktivem Füllstoff (="Schermaterial") und 0.1-20 % w/w Polymerpartikel (mit Abmessungen Schermaterial >1 µm zu Polymerpartikel <1 µm 5:1 bis 2000:1). Die Methode besteht aus den Schritten: (a) Dispergieren des Materials unter intensivem Mischen zu einer teigigen Masse; (b) Ausrollen der Masse zu einer Matte; und (c) Falten der Matte und Auswalzen im Winkel 45-135° bezogen auf die vorherige Richtung, wobei überschüssiges Lösungsmittel vor oder nach dem Mischschritt entfernt wird und alle Schritte bei 15-40°C ausgeführt werden. Das Verbundmaterial kann als Adsorptionsfolie für Gase und Flüssigkeiten verwendet werden.

EP 0659469 beschreibt eine Membran bestehend aus PTFE und Zeolith A zur Trennung von Flüssigkeiten, z.B. Wasser/Ethanol mittels Pervaporation oder Dampfpermeation. Die Membran wird auf einem porösen Träger abgeschieden. Der verwendete hydrophile Zeolith hat ein Verhältnis SiO₂:Al₂O₃ von 2-6:1.

JP 2010000435 beschreibt eine Kompositmembran bestehend aus Fluorpolymer (= poröser Träger, z.B. PTFE) und kristallinem Zeolithen, welcher nach Oberflächenbehandlung (Metallierung oder Hydrophilisierung) des Trägers in einem hydrothermalen Verfahren auf den Träger aufgebracht wird, zur Trennung von Flüssigkeiten (z.B. Wasser/Ethanol) oder Entzug von Restwasser aus >95% Ethanol. Die feste Verankerung des Zeolithen auf der Fluorpolymer-Oberfläche wird trotz der eigentlichen Inkompatibilität der beiden Substanzen erfindungsgemäß erreicht und damit die Beständigkeit der Kompositmembran verbessert. Ergänzend beschreibt JP 2010115610 das Einbringen von hochmolekularen Polymeren (Silikonkautschuk, Polyvinylalkohol, Polyacrylamid, Polyethylenglykol, Polyimid oder Polyamidimid oder seiner Carbide) in Defektstellen der kristallinen Zeolith-Oberfläche zur Verbesserung der Trennleistung o.g. Komposite.

US20100038316 beschreibt einen Verbundkörper aus PTFE und Zeolith, der dadurch entsteht, dass auf eine PTFE-Lage ein Binder und/oder Zeolith aufgebracht wird und daher beide Bestandteile miteinander schichtweise verbunden ("bonded"=verklebt) werden. Es entstehen dadurch mindestens 2, höchstens 3 Schichten, die zusammen eine Membran bzw. einen Film aus den Bestandteilen PTFE, Kleber und Zeolith ergeben. Der Kleber (oder Binder) kann z.B. Polyvinylalkohol sein, welcher in einem geeigneten Lösungsmittel gelöst, mit dem Zeolith vermischt und anschließend auf die PTFE-Lage aufgetragen wird. Der Zeolith kann auch so verändert sein, dass er katalytisch wirksam ist, z.B. durch Metallionen.

WO 07104354 beschreibt einen Packungsaufbau für die Säulenchromatographie (z.B. HPLC), welcher für die Auftrennung verschiedener Komponenten aus einem Fluid verwendet wird. Die Füllung besteht aus elastischem Polymer-Netzwerk, in welchem das eigentliche Füllmaterial eingelagert ist. Das elastische Netzwerk besteht aus mindestens einem der folgenden Stoffe: organisches oder anorganisches Material, Polymer, Gummi, Kautschuk, PTFE, expandiertes PP etc., das eigentliche Füllmaterial wiederum besteht z.B. aus Zeolith und/oder PTFE. Es wird außerdem ein Auflage-Element beansprucht, das das Füllmaterial umgeben kann oder sich oberhalb, unterhalb oder auf beiden Seiten des Füllmaterials befinden kann. Das Stützelement kann ein Edelstahl-Drahtsieb, Gewebe, Sinterkörper oder Filter sein. Die Verwendung eines Drahtsiebes wird als vorteilhaft bei der Entfernung von verdichtetem Packungsmaterial am Kopf der HPLC-Säule, sowie bei der Fertigung der Säule beschrieben.

EP 0 773 829 beschreibt Kompositmembranen aus fibrilliertem PTFE oder geblasenen Mikrofasern (Polyamid, Polyester, Polyurethan, Polyolefin etc.), in die unter Zuhilfenahme eines flüssigen Gleitmittels zur Vereinfachung der Verarbeitbarkeit hydrophobes Molsieb mit Porendurchmesser 5,5-6,2 Ångstrom im Verhältnis 40:1 bis 1:40 als selektives Sorptionsmedium ausschließlich zur Festphasenextraktion oder chromatographischen Trennung eingearbeitet ist. Die teigartige Masse wird biaxial kalandriert, wodurch sich schließlich nach dem Trocknen ein poröser Film ergibt, dessen Porosität von der Menge an Gleitmittel abgeleitet werden kann. Unter Festphasenextraktion ist der physikalische Trennprozess zwischen einer fluiden und einer festen Phase zu verstehen, wobei die zu isolierende (= analysierende) Komponente in einem Lösungsmittel gelöst ist. Es wird ferner innerhalb des Verfahrens ein thermischer Desorptionsschritt der entstandenen adsorbierten organischen Komponente beansprucht. In o.g. Patent werden u.a. die Patente US 4153661, US 4460642 und US 5071610 des gleichen Patentinhabers zitiert, die in analoger Weise poröse faserförmige Membranen auf Basis von PTFE zur Einarbeitung von Sorbentien oder katalytisch wirksamen partikelförmigen Stoffen beschreiben.

Die vorliegende Erfindung soll ein Material zur Verfügung stellen, welches geeignet ist, organische Moleküle aus Fluiden (d.h. Gasen und Flüssigkeiten) zu adsorbieren, und dabei eine verbesserte Materialstabilität und erhöhte Standzeit in technischen Säulenpackungen, sowie eine hohe Adsorptionsleistung für die organischen Zielmoleküle, d.h. eine hohe Adsorptionskapazität, also die Fähigkeit, eine hohe Masse organischer Zielmoleküle pro Masseneinheit Adsorptionsmaterial zu adsorbieren, bei gleichzeitig niedriger Wasseradsorption aufweist. Von besonderer Bedeutung für die technische Anwendung sind ein möglichst niedriger Druckverlust durch die Anordnung des erfindungsgemäßen Materials in einem Fluidstrom, sowie gegenüber den im Stand der Technik beschriebenen Materialien verbesserte thermische Eigenschaften.

Zudem soll die Erfindung ein Verfahren zur Abtrennung von organischen Molekülen aus einem gasförmigen oder flüssigen Stoffgemisch zur Verfügung stellen.

Diese Aufgabe wird durch die im Folgenden beschriebene Erfindung gelöst.

### Beschreibung der Erfindung

In einem ersten Aspekt stellt die Erfindung ein Verbundmaterial bereit bestehend aus
a) einer porösen Matrix aus einem fluorhaltigen Polymer mit einem Anteil von Tetrafluorethylen-Monomereinheiten von wenigstens 95 mol-% bezogen auf die gesamte Anzahl der Monomoereinheiten;
b) hydrophoben Zeolith-Partikeln, welche in die Matrix eingebettet und von ihr umgarnt sind, und
c) mindestens einem metallischen Werkstoff;
d) optional wenigstens einer weiteren Komponente,
   wobei
   die Menge des metallischen Werkstoffs c) 1 bis 90 Gew.-% bezogen auf die Summe des Gewichts aller Bestandteile beträgt,
   das Verhältnis des Gewichts der Komponente a) zu der Summe des Gewichts der Komponenten b) und d) 2:98 bis 30:70 beträgt, und
   das Verhältnis des Gewichts der Komponente b) zum Gewicht der Komponente d) 80:20 bis 100:0 beträgt.

In einem zweiten Aspekt stellt die Erfindung ein Verfahren bereit, das die Schritte
a) Inkontaktbringen eines Stoffgemisches enthaltend wenigstens eine organische Komponente mit dem Verbundmaterial gemäß dem vorstehend beschriebenen ersten Aspekt, so dass die wenigstens eine organische Komponente an dem Verbundmaterial adsorbiert wird und ein beladenes Verbundmaterial erhalten wird;
b) Trennen des Stoffgemischs und des beladenen Verbundmaterials;
c) Desorbieren der wenigstens einen organischen Komponente von dem beladenen Verbundmaterial
umfasst.

Bevorzugte Ausführungsformen werden nachstehend beschrieben und in den abhängigen Ansprüchen definiert.

### - Erfindungsgemäßes Verbundmaterial

Nachfolgend werden die Komponenten a), b), c) und d) des erfindungsgemäßen Verbundmaterials erläutert.

Das erfindungsgemäße Verbundmaterial umfasst ein fluorhaltiges, fibrillierbares Polymer, bevorzugt PTFE, und einen hydrophoben Zeolithen, der zur Adsorption kleiner organischer Moleküle aus einem wässrigen Fluid geeignet ist. Zur Versteifung des Materials ist Metall in Form von Metallgitter, -gewebe, -netz, gelochten oder gestanzten Metallplatten eingearbeitet. Es wurde überraschend gefunden, dass durch die Einarbeitung von Metall neben der mechanischen Stabilisierung eine nicht unerhebliche positive Änderung der thermischen Eigenschaften des Materials erreicht wird.

### a) Matrix aus fluorhaltigem Polymer

Die Matrix des Verbundmaterials besteht aus fluorhaltigem Polymer, d.h. einem Homo- oder Copolymer mit einem Anteil von Tetrafluorethylen-Monomereinheiten von wenigstens 95 mol-%. Das fluorhaltige Polymer ist fibrillierbar und kann durch Fibrillierung eine poröse Matrix bilden. Das fluorhaltige Polymer ist zudem chemisch inert und in Gegenwart von Wasser oder organischen Molekülen nicht quellbar. Vorzugsweise umfasst das fluorhaltige Polymer einen Anteil von Tetrafluorethylen-Monomereinheiten von wenigstens 99 mol-%.

Als Beispiele für das fluorhaltige Polymer können Polytetrafluorethylen (PTFE), Tetrafluorethylen-Hexafluorpropylen-Copolymer, Tetrafluorethylen-Chlortrifluorethylen-Copolymer, Tetrafluorethylen-Perfluor-(2,2-dimethyl-1,3-dioxol)-Copolymer und Tetrafluorethylen-Perfluor(C₁₋₆-alkylvinylether)-Copolymer wie etwa Tetrafluorethylen-Perfluor(butenylvinylether)-Copolymer genannt werden. PTFE ist bevorzugt.

Das Polymer kann als Pulver oder Dispersion eingesetzt werden. Bevorzugt werden tensidfreie PTFE-Pulver verwendet, denn das Nichtvorhandensein von oberflächenaktiven Substanzen, die für die Haltbarkeit von PTFE-Dispersionen notwendig sind, eliminiert die unerwünschten Effekte der Reduktion der verfügbaren Zeolith-Oberfläche bzw. Erhöhung der Wasseradsorption durch ebensolche Tenside.

Diese Polymere sind gemäß den in EP 0 773 829 B1 (und darin zitierten Dokumenten des Stands der Technik) beschriebenen Verfahren fibrillierbar, so dass eine poröse und faserige Matrix gebildet wird.

### b) Hydrophobe Zeolith-Partikel

Für das erfindungsgemäße Material sind insbesondere jene Sorbentien interessant, die geeignet sind, organische polare Moleküle aus wasserenthaltenden Fluiden zu sorbieren und unter geeigneten Bedingungen wieder zu desorbieren, um sie anzureichern oder aufzureinigen.

Geeignet sind insbesondere hydrophobe Zeolithe, d.h. Zeolithe mit einem molaren SiO₂:Al₂O₃-Verhältnis größer 100:1, bevorzugt größer 200:1, mehr bevorzugt größer 500:1. Diese Zeolithe sind für die Adsorption von organischen Molekülen wie Alkoholen (z.B. Ethanol, Butanol), Ethern, Ketonen (z.B. Aceton), Aldehyden (z.B. Acetaldehyd), Carbonsäuren (z.B. Essigsäure) und Carbonsäureestern (z.B. Ethylacetat) etc. generell gut geeignet. Die Bestimmung des SiO₂:Al₂O₃-Verhältnisses erfolgt durch Röntgenfluoreszenzspektroskopie (XRF) einer bei 100 °C eine Stunde lang getrockneten Probe, die anschließend mit einem Bindemittel zu einer Tablette gepresst wird, durch Bestimmung des molaren Verhältnisses von Si:Al, das zum molaren Verhältnis SiO₂:Al₂O₃ umgerechnet wird.

Um besonders gute Adsorptionseigenschaften aufzuweisen, d.h. eine große Anzahl organischer Moleküle pro Gewichtseinheit Zeolith adsorbieren zu können, sollten die Zeolithe eine große, durch die BET-Methode bestimmte Oberfläche pro Gewichtseinheit aufweisen. Für die vorliegende Erfindung geeignete Zeolithe weisen eine Oberfläche lt. BET-Methode von 150 m²/g oder größer, bevorzugt 200 m²/g oder größer, noch bevorzugter von 300 m²/g oder größer auf.

Die Oberfläche wird mit einem vollautomatischen Stickstoffporosimeter der Firma Micromeritics Typ ASAP 2010 unter Verwendung von Stickstoff als adsorbiertem Gas gemäß dem folgenden Verfahren nach DIN 66131 (Juli 1993) bestimmt. Die Probe wird im Hochvakuum auf die Temperatur von flüssigem Stickstoff abgekühlt. Anschließend wird kontinuierlich Stickstoff in die Probenkammern dosiert. Durch die Erfassung der adsorbierten Gasmenge als Funktion des Druckes wird bei konstanter Temperatur eine Adsorptionsisotherme ermittelt. In einem Druckausgleich wird das Analysengas schrittweise entfernt und eine Desorptionsisotherme aufgenommen. Zur Ermittlung der spezifischen Oberfläche und der Porosität nach der BET-Theorie werden die Daten gemäß DIN 66131 (Juli 1993) ausgewertet.

Unter diesen Gesichtspunkten bevorzugt sind Zeolithe der Typen Silicalit, ß-Zeolith, Mordenit, Y-Zeolith, MFI-Zeolith, Ferrierit (FER-Zeolith), dealuminierter, ultrastabiler Zeolith Y (USY-Zeolith) und Erionit (ERI-Zeolith). Das erfindungsgemäße Verfahren lässt auch Mischungen dieser Zeolithe zu.

Es werden vorzugsweise Zeolith-Partikel der Teilchengröße (d₅₀) von 0,5 bis 100 µm, weiter bevorzugt von 1 bis 50 µm und besonders bevorzugt von 5 bis 25 µm eingesetzt. Grundsätzlich nimmt mit abnehmender Teilchengröße die spezifische Oberfläche, d.h. die Oberfläche pro Masseneinheit zu. Eine große spezifische Oberfläche führt in der Regel zu einer hohen und damit vorteilhaften Adsorptionsgeschwindigkeit. Da die Handhabung und Verarbeitung eines Pulvers jedoch mit abnehmender Teilchengröße zunehmend schwierig und aufwendig wird, wäre es nicht vorteilhaft, sehr geringe Teilchengrößen zu wählen, wenngleich dies prinzipiell möglich ist.

Es kann ein einzelner Zeolith-Typ oder eine Mischung aus mehreren Zeolith-Typen eingesetzt werden. Der einzelne Zeolith-Typ oder die Zeolith-Typen können in einer einheitlichen Teilchengröße oder in mehreren Teilchengrößen eingesetzt werden.

### c) Metallischer Werkstoff

Für das erfindungsgemäße Verbundmaterial eignen sich metallische Werkstoffe, d.h. reine Metalle und Legierungen, die in Gegenwart von Wasser und organischen Molekülen chemisch inert sind, d.h. nicht oder nur eingeschränkt mit Wasser und/oder organischen Verbindungen reagieren. Eingeschränktes Reagieren mit Wasser und/oder organischen Verbindungen bedeutet beispielsweise, dass eine Passivierung der Oberfläche des metallischen Werkstoffs auftritt, aber keine chemische Reaktion, die schließlich zum vollständigen Abbau des metallischen Werkstoffs führt.

Unter diesen Gesichtspunkten bevorzugt sind korrosionsfreie Metalle, besonders bevorzugt Edelstähle, die in der Lebensmittel- und chemischen Industrie verwendet werden, z.B. X2CrNi1911 (Werkstoffnummer = WNr. 1.4306), X12CrNi177 (WNr. 1.4310), oder X5CrNi1810 (WNr. 1.4301).

Die Form, in der der metallische Werkstoff in dem Verbundmaterial vorliegt, ist nicht beschränkt. Beispielsweise kann der metallische Werkstoff in flächiger Form, d.h. beispielsweise in Form von Metallgittern, -geweben, -netzen, sowie gelochten oder gestanzten Metallplatten oder -blechen, oder in Teilchenform, d.h. beispielsweise in Form von Pulvern oder Spänen, in dem Verbundmaterial vorliegen. Durch die als Beispiele genannten Strukturen wird sichergestellt, dass eine gute Verbindung zwischen Metall und Verbundmaterial erzielt wird. Der metallische Werkstoff kann in mehreren Formen in dem Verbundmaterial vorliegen, d.h. sowohl in Teilchenform als auch in flächiger Form.

Bei der Verwendung des metallischen Werkstoffs in flächiger Form wird eine Maschenweite bzw. Lochöffnung von 0,5-5 mm, insbesondere von 1-2 mm bevorzugt. Die Anzahl und Verteilung von Löchern pro Flächeneinheit ist nicht sonderlich beschränkt und wird durch Überlegungen des Fachmanns hinsichtlich der gewünschten Durchlässigkeit und der Stabilität bestimmt. Ebenso ist die Dicke des metallischen Werkstoffs in der eingesetzten flächigen Form nicht sonderlich beschränkt, sofern die gewünschte Dimensionsstabilität erreicht wird. Zu diesem Zweck beträgt die Dicke des metallischen Werkstoffs gängigerweise 0,1-1 mm, bevorzugt 0,2-0,5 mm besonders bevorzugt 0,25 mm.

In dem erfindungsgemäßen Verbundmaterial beträgt die Menge des metallischen Werkstoffs c) 1 bis 90 Gew.-% bezogen auf die Summe aller Bestandteile des Verbundmaterials. Vorzugsweise beträgt die Menge des metallischen Werkstoffs c) 5 bis 80 Gew.-%, weiter bevorzugt 10 bis 70 Gew.-%, am meisten bevorzugt 15 bis 65 Gew.-%, bezogen auf die Summe aller Bestandteile des Verbundmaterials.

### d) Weitere Komponente

In dem erfindungsgemäßen Verbundmaterial können optional eine oder mehrere Komponenten vorhanden sein, die beispielsweise aus Hilfsstoffen, Tensiden, Gleitmitteln, Fällungskieselsäure, Silica, Aktivkohle, Pigmenten, Glaskugeln oder -fasern, Kunststofffasern, Fasern natürlichen Ursprungs, Tonmineralen wie etwa Bentonit ausgewählt sein können.

Das fluorhaltige Polymer a) liegt in einem Verhältnis zum Gesamtgewicht der hydrophoben Zeolith-Partikel b) und der optional vorhandenen weiteren Komponente d) von 2:98 bis 30:70, vorzugsweise von 4:96 bis 20:80, weiter bevorzugt von 5:95 bis 15:85.

Das Verhältnis des Gewichts der hydrophoben Zeolith-Partikel b) zum Gewicht der Komponente d) beträgt 80:20 bis 100:0, d.h. die Komponente d) ist optional. Vorzugsweise beträgt das Verhältnis des Gewichts der hydrophoben Zeolith-Partikel b) zum Gewicht der Komponente d) 90:10 bis 100:0, weiter bevorzugt 95:5 bis 100:0.

In einer bevorzugten Ausführungsform beträgt das Verhältnis des Gewichts des fluorhaltigen Polymers a) zum Gesamtgewicht der hydrophoben Zeolith-Partikel b) und der optional vorhandenen weiteren Komponente d) in einem Bereich von 4:96 bis 20:80, weiter bevorzugt 5:95 bis 15:85, wobei das Verhältnis des Gewichts der hydrophoben Zeolith-Partikel b) zum Gewicht der Komponente d) 90:10 bis 100:0 beträgt.

### - Herstellung des erfindungsgemäßen Verbundmaterials

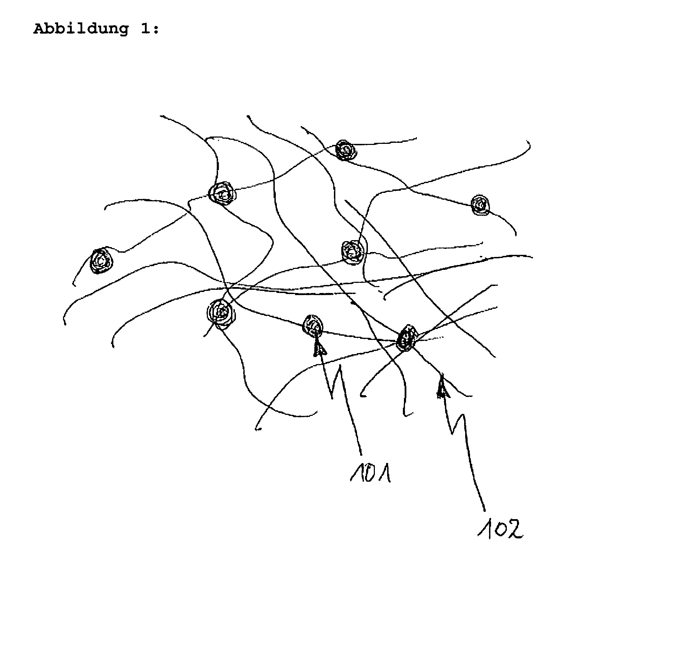

Das Verbundmaterial wird durch Vermischen der Komponenten a), b), der optionalen weiteren Komponente(n) d) und des metallischen Werkstoffs c), sofern der metallische Werkstoff c) in einer geeigneten kleinteiligen Form, also beispielsweise in Pulverform, verwendet wird, in den oben genannten Mengen und anschließendem Kneten hergestellt, wobei sich unter Scherung die Fibrillierung des Polymers und Einarbeitung des Zeolithen in die poröse Polymermatrix einstellt [Abbildung 1]. Das Kneten wird bei Raumtemperatur oder vorzugsweise bei einer erhöhten Temperatur wie etwa 30 °C oder mehr, 50 °C oder mehr oder 70 °C oder mehr durchgeführt, da bei einer Temperatur in diesen Bereichen in der Regel eine bessere Verarbeitbarkeit und insbesondere eine bessere Fibrillierung des fluorhaltigen Polymers möglich ist. Die Temperaturobergrenze wird in erster Linie durch thermische Stabilität der in der Mischung enthaltenen Komponenten bestimmt. Unter diesem Gesichtspunkt ist in der Regel eine Verarbeitung bei einer Temperatur von nicht mehr als 200 °C bevorzugt, weiter bevorzugt von nicht mehr als 150 °C.

Um eine gute Mischbarkeit der Komponenten des Verbundmaterials zu erreichen, werden Polymer a) und Zeolith b) bevorzugt in Pulverform eingesetzt. Das Polymer a) kann beispielsweise auch in Form einer handelsüblichen Dispersion in Wasser eingesetzt werden. Solche handelsüblichen Dispersionen können Hilfsstoffe wie etwa Stabilisatoren, Tenside oder andere, die Oberflächenspannung verändernde Komponenten und/oder andere Hilfsstoffe enthalten.

Um den Misch- und Scherprozess zu erleichtern, kann als Gleitmittel Wasser oder Alkohol zugesetzt werden. Um später auf einen energie- und kostenintensiven Trockenschritt weitgehend verzichten zu können, wird aber bevorzugt mit möglichst wenig Flüssigkeit gearbeitet, d.h. außer der über die PTFE-Dispersion (maximal 40% der Dispersion) zugeführten Flüssigkeitsmenge wird kein Gleitmittel zugesetzt.

Nach dem Knetschritt wird das teig- bis vliesartige Produkt zwischen beheizten Walzen (Temperatur 60-150 °C) in mehreren Schritten biaxial zunächst zu einer Matte, dann zu einem Film ausgewalzt, wobei die Fibrillierung optimiert und eine homogene End-Schichtdicke von 0,3 bis 1 mm, bevorzugt 0,4-0,6 mm eingestellt wird. Geeignet für diesen Schritt ist ein beheizbares Kalander- oder Rollwalzsystem aus mindestens 2 Rollen, bevorzugt 4 Rollen oder mehr.

Ein geeignetes Verfahren zur Herstellung eines Verbundmaterials aus einem Polymer a) und einem Zeolith b) wird auch in EP 0 773 829 B1 und darin zitierten Dokumenten beschrieben.

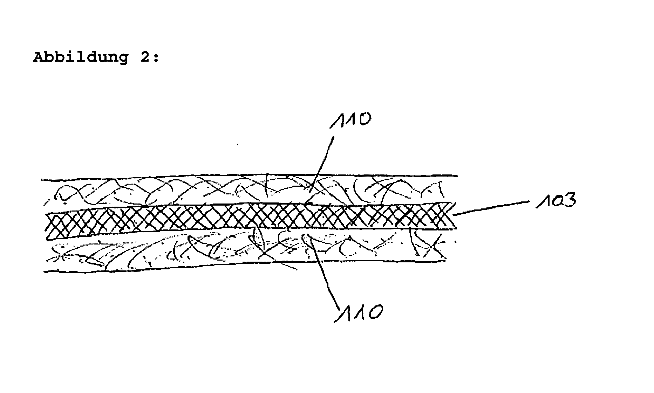

Wenn ein metallischer Werkstoff in einer flächigen Form eingebracht werden soll, wird das so erhaltene Material in einem oder mehreren Schritten zwischen druckbelasteten Walzen in einem Laminator oder Kalander so mit dem metallischen Werkstoff in flächiger Form, z.B. Edelstahlgeflecht, verpresst, dass ein Verbund aus mindestens einer Lage des Materials und dem metallischen Werkstoff gebildet wird. Bevorzugt wird eine Lage des metallischen Werkstoffs zwischen zwei Lagen des Materials eingeschlossen [Abbildung 2]. Bevorzugt durchdringen sich beide Schichten des Materials durch die Öffnungen in dem flächigen metallischen Werkstoff hindurch, wodurch die Stabilität des Verbundes optimiert wird. Der Schritt des Verbindens des metallischen Werkstoffs und des Materials kann bei Raumtemperatur, vorteilhaft aber bei 70-250°C geschehen, um Restfeuchte zu vertreiben, die beispielweise aufgrund der Verwendung von Wasser als Gleitmittel beim Mischen und/oder Kneten von fluorhaltigem Polymer a) und Zeolith-Partikeln b) wie oben beschrieben in dem Material vorliegen kann. Optional schließt sich ein Trocknungsschritt an.

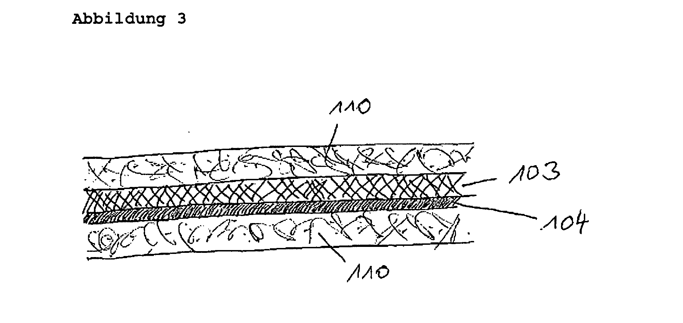

Optional wird ein oder mehrere Heizelement(e) in das Material so eingebracht, dass die Wärmeenergie vom Heizelement an das metallische Material gut übertragen werden kann. Der metallische Werkstoff kann ggf. selbst die Funktion des Heizelementes übernehmen, z.B. durch Beheizung mittels magnetischer Induktion, elektrische Widerstandsheizung oder Wärmeaustausch. Durch das Heizelement kann die Adsorptionsund Desorptionstemperatur im Sinne der Prozessausbeute optimiert werden. Es dient außerdem zur Erleichterung der ggf. notwendigen Regeneration des Materials [Abbildung 3].

Das Verbundmaterial gemäß dem ersten Aspekt der Erfindung kann zur Adsorption von organischen Molekülen verwendet werden, die in einem gasförmigen oder flüssigen Stoffgemisch enthalten sind.

### - Verfahren zur Adsorption organischer Moleküle

Nachstehend wird das Verfahren gemäß dem zweiten Aspekt der Erfindung beschrieben.
a) Inkontaktbringen eines Stoffgemischs, das wenigstens einen Typ organischer Moleküle enthält, mit dem Verbundmaterial gemäß dem ersten Aspekt der vorliegenden Erfindung.

Die Adsorption mindestens einer organischen Komponente, d.h. mindestens eines Typs organischer Moleküle, erfolgt aus einem Fluid, d.h. aus einem flüssigen oder gasförmigen Stoffgemisch, das wenigstens eine organische Komponente und bevorzugt Wasser enthält. Als nicht-organische Komponenten können auch Schwefelwasserstoff, Ammoniak, Wasserstoff, Kohlendioxid oder Stickstoff in dem Fluid vorliegen.

Bei der wenigstens einen organischen Komponente handelt es sich beispielsweise um eine Substanz aus einer der Substanzklassen Alkohole (z.B. Ethanol, Butanol), Ether (z.B. Methyl-tert-butylether oder Tetrahydrofuran) Ketone, (z.B. Aceton), Aldehyde (z.B. Acetaldehyd), Carbonsäuren (insbesondere C₁₋₄-Carbonsäuren wie z.B. Essigsäure oder Propionsäure) und Carbonsäureester (z.B. Ethylacetat). Diese Substanzen werden bevorzugt fermentativ oder enzymatisch hergestellt. Besonders bevorzugt handelt es sich bei dieser Herstellung um eine ethanolische Gärung mittels Hefen oder Bakterien oder um eine sogenannte ABE-Fermentation mittels Bakterien, wobei letztere Aceton, Butanol und Ethanol (ABE) produzieren.

Bevorzugt handelt es sich bei dem Fluid, das für die Adsorption verwendet wird, um ein gasförmiges Stoffgemisch, das durch Gas-Stripping einer wässrigen Lösung mit flüchtigen organischen Verbindungen der oben genannten Substanzklassen erhalten worden ist. Besonders bevorzugt handelt es sich bei der wässrigen Lösung um eine Fermentationslösung, ganz besonders bevorzugt um eine Fermentationslösung der oben genannten Fermentationsprozesse. Dieses Gas-Stripping wird besonders bevorzugt in-situ durchgeführt, wobei in-situ bedeutet, dass das Gas-Stripping während der Fermentation erfolgt. Das Gas-Stripping kann aber auch nach abgeschlossener Fermentation erfolgen. Das Gas-Stripping kann in einer mit dem Fermenter verbundenen externen Gas-Stripping-Vorrichtung erfolgen.

Das Verbundmaterial gemäß dem ersten Aspekt der vorliegenden Erfindung wird mit dem Stoffgemisch in Kontakt gebracht, so dass eine Adsorption der wenigstens mindestens einen organischen Komponente erfolgen kann. Beispielsweise kann das Verbundmaterial in einen Strom des gasförmigen oder flüssigen Stoffgemischs so eingebracht werden, dass das Stoffgemisch über die Oberfläche des Verbundmaterials strömen kann, beispielsweise durch Anordnen des Verbundmaterials in einer geeigneten geometrischen Form in einer Säule, durch den ein Strom des Fluids geführt wird.

Durch Adsorption des wenigstens einen Typs organischer Moleküle an dem Verbundmaterial wird das Verbundmaterial mit dem wenigstens einen Typ organischer Moleküle beladen, so dass ein beladenes Verbundmaterial erhalten wird.
b) Das beladene Verbundmaterial wird von dem Stoffgemisch getrennt, nachdem das Verbundmaterial über einen Zeitraum, der für die Adsorption der wenigstens einen organischen Komponente ausreichend lang ist, mit dem Stoffgemisch in Kontakt gewesen ist, um eine ausreichende Beladung des Verbundmaterials zu erreichen. Wenn ein Strom des Fluids über die Packung des Verbundmaterials geführt wird, so dass Adsorption wenigstens einer darin enthaltenen organischen Komponente eintritt, bildet sich in der Regel ein Gradient der Beladung, d.h der Konzentration der an dem Verbundmaterial adsorbierten organischen Komponente, in Strömungsrichtung aus. Das bedeutet, dass die Konzentration der an dem Verbundmaterial adsorbierten organischen Komponente in den stromaufwärts liegenden Bereichen des Verbundmaterials in der Regel höher ist als in den weiter stromabwärts liegenden Bereichen des Verbundmaterials.
c) Die wenigstens eine organische Komponente wird durhc Desorption von dem Verbundmaterial getrennt.

Die Desorption kann
(a) durch Verdrängung mittels anderer Komponenten;
(b) thermisch, d.h. durch Erhöhung der Temperatur des Adsorptionsmittels (Temperature-Swing-Adsorptionsverfahren (TSA));
(c) durch das sogenannte Pressure-Swing-Adsorptionsverfahren (PSA), d.h. durch Absenkung des Druckes;
(d) durch eine Kombination der in (a) bis (c) genannten Verfahren
erfolgen.

In einer bevorzugten Ausführungsform des erfindungsgemäßen Verfahrens wird bei der Desorption ein Spülgas verwendet. Bevorzugte Spülgase sind inerte Gase, besonders bevorzugt sind die Spülgase Luft, Kohlenstoffdioxid, Stickstoff, Edelgase oder Mischungen daraus. In einer weiteren Ausführungsform des erfindungsgemäßen Verfahrens enthält das Spülgas Wasser. Besonders bevorzugt liegt die Temperatur des Spülgases oberhalb der Temperatur des Verbundmaterials.

Bevorzugt ist die Strömungsrichtung bei der Desorption umgekehrt zur Strömungsrichtung des Fluids bei der Adsorption d.h. so dass die Desorption entgegen dem bei der Adsorption entstandenen Gradienten der Konzentration der an dem Verbundmaterial adsorbierten organischen Komponente erfolgt.

### Kurze Beschreibung der Abbildungen

Abbildung 1 ist eine schematische Darstellung eines Verbundmaterials aus PTFE und Zeolith (101 = Zeolithpartikel, 102 = netzartige PTFE-Fibrillen).

Abbildung 2 ist eine schematische Darstellung einer Ausführungsform des erfindungsgemäßen Verbundmaterials, bestehend aus 103 = metallischem Werkstoff, 110 = PTFE-Zeolith-Komposit gemäß Abb. 1.

Abbildung 3 ist eine schematische Darstellung einer Ausführungsform des erfindungsgemäßen Verbundmaterials, bestehend aus 103 = metallischem Werkstoff, 110 = PTFE-Zeolith-Komposit gemäß Abb. 1, 104 = Heizelement.

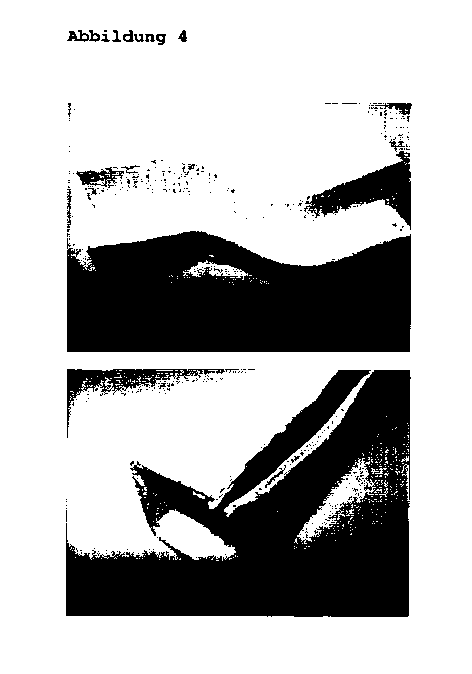

Abbildung 4 zeigt beispielhaft zwei Ausführungsformen der erfindungsgemäßen Schicht-Formkörper gemäß Abb. 2.

### Beispiele

Das erfindungsgemäße Material wird anhand folgender, nicht einschränkender, Beispiele erläutert:
Beispiel 1: Herstellung eines PTFE-Zeolith-Verbundmaterials (nicht erfindungsgemäß)
   25,1 g PTFE-Dispersion TE3893-N (ca. 60% PTFE Gehalt, DuPont) werden mit 150 g getrocknetem Zeolith (ZSM-5, H-Form; SiO₂/Al₂O₃ > 800; Hersteller: Süd-Chemie AG, Deutschland) vermischt (entspricht 9% w/w PTFE) und anschließend 45 Minuten in einem Werner&Pfleiderer LUK 075 Laborkneter bei 90°C geknetet, wobei sich unter Scherung die Fibrillierung des PTFE und Einarbeitung des Zeolithen einstellt.
   Nach dem Knetschritt wird das vliesartige Produkt in einem Fetzel Kalandersystem zwischen beheizten Walzen (Temperatur 70 °C) in mehreren Schritten biaxial zu einem Film mit einer Schichtdicke von 0,5 mm ausgewalzt, wobei die Fibrillierung optimiert wird.
Beispiel 2: Herstellung eines verstärkten PTFE-Zeolith-Verbundmaterials
   Eine Lage Edelstahlgewebe 20x30cm (Material 1.431, Drahtstärke 0,25mm, Maschenweite 1,00 mm; Fa. Drahtweberei Gräfenthal GmbH, Gräfenthal) wird zwischen zwei Lagen filmartiges Material aus Beispiel 1 (Dicke 0.5mm, Abmessungen 20x30cm) gebracht und die drei Lagen in einem Laminator der Fa. Fetzel (Walzenspalt 0,5mm; Vorschub 1-1,4m/min) bei 60-70°C in zwei Durchgängen verpresst.
Beispiel 3: Adsorption von Ethanol aus einem Gasstrom
   500 mL einer 5%(w/v)-Ethanol-Wasser-Lösung wurden für 24 Stunden mit einem Volumenstrom von 5,5 L/min gestrippt. Mit Hilfe eines Wasserbades (Heizplatte Firma IKA; RCT basic, Temperaturfühler Firma VWR; Typ VT5) wurde die Lösung auf 30°C temperiert. Es wurde eine Membranpumpe (KNF-Neuberger, Deutschland; Typ: N86KT.18) und eine Gaswaschflasche (VWR, Deutschland) verwendet. Der Gasstrom wurde durch eine Glassäule (Firma Gassner-Glastechnik, Deutschland; Innendurchmesser: 6,3 cm; Gesamtlänge 36 cm) geleitet, welche mit 155,93 g des Verbundmaterials aus Beispiel 2 in gerollter Form gepackt war. Der Gasstrom wurde im Rahmen einer Kreislaufführung in die Gaswaschflasche rückgeführt, so dass das System geschlossen war. Die Glassäule wurde in einem Klimaschrank (Firma: Vötsch Industrietechnik, Deutschland; VC 4043) bei einer Temperatur von 40 °C gehalten.

Nach 24 Stunden wurde ein Ende der Glassäule geschlossen und das andere Ende über Teflonschläuche an zwei Kühlfallen in Dewargefäßen (Firma: KGW Isotherm, Deutschland), die über flüssigen Stickstoff gekühlt wurden, und schließlich an eine Vakuumpumpe (Firma: VacuuBrand, Deutschland; Typ: CVC3000) angeschlossen. Die Glassäule wurde aus dem Klimaschrank genommen und mit Isoliermaterial ummantelt. Die Desorption startete sobald die Säule luftdicht mit dem Desorptionsaufbau verbunden war. Die Desorptionszeit betrug 20 Minuten bei 50 mbar Absolutdruck. Nach der Desorptionszeit wurde der Versuch gestoppt. Das Volumen des kondensierten Desorbats wurde bestimmt und die Ethanol-Konzentration gaschromatografisch gemessen.

## Patentansprüche

1. Verbundmaterial bestehend aus
a) eine poröse Matrix aus einem fluorhaltigen Polymer mit einem Anteil von Tetrafluorethylen-Monomereinheiten von wenigstens 95 mol-% bezogen auf die gesamte Anzahl der Monomereinheiten;
b) hydrophobe Zeolith-Partikel, welche in die Matrix eingebettet und von ihr umgarnt sind, und
c) mindestens einen metallischen Werkstoff;
d) optional wenigstens einer weiteren Komponente,
wobei
die Menge des metallischen Werkstoffs c) 1 bis 90 Gew.-% bezogen auf die Summe des Gewichts aller Bestandteile beträgt,
das Verhältnis des Gewichts der Komponente a) zu der Summe des Gewichts der Komponenten b) und d) 2:98 bis 30:70 beträgt, und
das Verhältnis des Gewichts der Komponente b) zum Gewicht der Komponente d) 80:20 bis 100:0 beträgt.

2. Verbundmaterial gemäß Anspruch 1, wobei das fluorhaltige Polymer Polytetrafluorethylen ist.

3. Verbundmaterial gemäß Anspruch 1 oder Anspruch 2, wobei das Verhältnis des Gewichts der Komponente a) zu der Summe des Gewichts der Komponenten b) und d) 4:96 bis 20:80 beträgt.

4. Verbundmaterial gemäß einem der Ansprüche 1 bis 3, wobei das Verhältnis der Komponente a) zu der Summe des Gewichts der Komponenten b) und d) 5:95 bis 15:85 beträgt.

5. Verbundmaterial gemäß einem der Ansprüche 1 bis 4, wobei die Menge des metallischen Werkstoffs c) 5 bis 80 Gew.-% bezogen auf die Summe des Gewichts aller Bestandteile beträgt.

6. Verbundmaterial gemäß einem der Ansprüche 1 bis 4, wobei die Menge des metallischen Werkstoffs c) 10 bis 70 Gew.-% bezogen auf die Summe des Gewichts aller Bestandteile beträgt.

7. Verbundmaterial gemäß einem der Ansprüche 1 bis 6, wobei der metallische Werkstoff ein Stahl mit der Werkstoffnummer 1.4301 ist.

8. Verbundmaterial gemäß einem der Ansprüche 1 bis 7, wobei der metallische Werkstoff in Form eines Drahtgewebes, eines Drahtgeflechts, einer mit Löchern versehenen Platte, in Form von Spänen oder in Pulverform vorliegt.

9. Verbundmaterial gemäß einem der Ansprüche 1 bis 8, wobei der metallische Werkstoff elektrisch, magnetisch-induktiv oder durch einen Wärmetauschprozess beheizbar ist.

10. Verbundmaterial gemäß einem der Ansprüche 1 bis 9, wobei der Zeolith eine Partikelgröße zwischen 0,5 und 100pm, aufweist.

11. Verbundmaterial gemäß einem der Ansprüche 1 bis 10, wobei der Zeolith aus der Gruppe bestehend aus Silicalit, ß-Zeolith, Mordenit, Y-Zeolith, MFI-Zeolith, Ferrierit (FER-Zeolith), dealuminiertem, ultrastabilem Zeolith Y (USY-Zeolith) und Erionit (ERI-Zeolith) und deren Mischungen ausgewählt ist.

12. Verbundmaterial gemäß einem der Ansprüche 1 bis 11, wobei der Zeolith ein SiO₂/Al₂O₃-Verhältnis von 100:1 oder größer aufweist.

13. Verbundmaterial gemäß einem der Ansprüche 1 bis 11, wobei der Zeolith ein SiO₂/Al₂O₃-Verhältnis von 200:1 oder größer aufweist.

14. Verwendung des Verbundmaterials gemäß einem der Ansprüche 1 bis 13 zur Adsorption von organischen Molekülen aus einem gasförmigen oder flüssigen Stoffgemisch enthaltend wenigstens eine organische Komponente.

15. Verfahren zur Abtrennung von organischen Molekülen aus einem gasförmigen oder flüssigen Stoffgemisch enthaltend wenigstens eine organische Komponente umfassend die Schritte
a) Inkontaktbringen eines Stoffgemisches enthaltend wenigstens eine organische Komponente mit dem Verbundmaterial gemäß einem der Ansprüche 1 bis 13, so dass die wenigstens eine organische Komponente an dem Verbundmaterial adsorbiert wird und ein beladenes Verbundmaterial erhalten wird;
b) Trennen des Stoffgemischs und des beladenen Verbundmaterials;
c) Desorbieren der wenigstens einen organischen Komponente von dem beladenen Verbundmaterial.

16. Verfahren gemäß Anspruch 15, wobei die Desorption in Schritt c) durch Verminderung des Drucks der das Verbundmaterial umgebenden Atmosphäre und/oder Erhöhung der Temperatur des Verbundmaterials bewirkt wird.
